# DEMANDE DE BREVET EUROPEEN

(11) **EP 2 147 709 A1**
(43) Date de publication de la demande: **27.01.2010**
(21) Numéro de dépôt: 09290461.4
(22) Date de dépôt: 17.06.2009
(51) Int. Cl.: B01D 15/00, C11B 3/10, C11C 3/10, G01N 21/53, C07C 67/56, B01J 49/00

(54) **Méthode de suivi du perçage de lit d'adsorbant dans un procédé de production d'esters alkyliques à partir d'huile végétale ou animale et d'un monoalcool aliphatique**

(30) Priorité: 22.07.2008 FR 0804153
(71) Demandeur: IFP, 92852 Rueil-Malmaison Cédex (FR)
(72) Inventeur: Koudil, Abdelhakim, 69001 Lyon (FR); Barthelet, Karin, 69003 Lyon (FR); Bournay, Laurent, 69440 Chaussan (FR)

(57) **Abrégé**

La présente invention décrit une méthode de suivi du perçage d'un lit d'adsorbants constitué par exemple de résines échangeuses d'ions, utilisé lors de l'étape de purification d'un flux d'esters contenant de la glycérine, par exemple obtenu dans un procédé de transestérification d'une huile végétale ou animale et d'un monoalcool aliphatique. Elle permet de détecter de façon instantanée à l'aide d'un turbidimètre placé en sortie du lit d'adsorbant la présence de glycérine dans le flux d'esters.

## Description

### DOMAINE DE L'INVENTION

L'invention concerne un procédé amélioré de purification d'un mélange d'esters et de glycérine.

### ART ANTÉRIEUR

En vue de leur utilisation comme biocarburant, les esters alkyliques d'huiles végétales sont produits à partir d'huiles végétales issues par exemple de colza, de tournesol, de soja ou même de palme. Mal adaptées à l'alimentation directe des moteurs diesel modernes des véhicules particuliers, les huiles végétales constituées essentiellement de triglycérides doivent être transformées par une réaction de transestérification avec un alcool, par exemple le méthanol ou l'éthanol, introduit en excès pour produire des esters méthyliques d'huiles végétales (EMHV) et de la glycérine.

La norme européenne actuelle sur les biocarburants EN 14 214 impose des teneurs maximales en méthanol, eau, glycérol libre ainsi qu'en mono-, di- et triglycérides : 0,2% masse pour le méthanol, 500 mg/kg pour l'eau, 0,02 % masse de glycérol libre, 0,8% masse en monoglycérides et 0.2% masse en di- et triglycérides.

Le glycérol libre, par opposition au glycérol lié, est défini comme étant une molécule de glycérol totalement détachée de toute chaîne carbonée et de formule C₃H₈O₃.

On parle de glycérol lié lorsque le groupement fonctionnel du glycérol C₃H₈O₃ se retrouve alkylé à une ou plusieurs chaînes d'acides gras donnant des molécules de monoglycérides, diglycérides ou triglycérides.

Les huiles végétales et/ou animales qui sont utilisées dans ce procédé de fabrication d'esters alkyliques peuvent être toutes huiles connues de l'homme du métier, par exemple de l'huile de colza, de palme, de tournesol, de soja, de copra, de ricin, ainsi que des corps gras d'origine animales comme le suif.

L'alcool mis en oeuvre est généralement un monoalcool aliphatique. De préférence, l'alcool est essentiellement constitué par du méthanol et/ou de l'éthanol.

Le glycérol est connu pour être infiniment soluble dans l'eau et les alcools, peu solubles dans les éthers et totalement insoluble dans le benzène (Handbook of Chemistry and Physics, 54th Edition 1973-1974), c'est-à-dire qu'il est soluble dans des milieux plutôt polaires. Les esters ne l'étant pas particulièrement, les esters méthyliques et le glycérol sont très peu solubles et le méthanol joue le rôle de co-solvant. Donc la teneur en glycérol dans la phase ester est d'autant plus importante que la température est élevée et que la teneur en méthanol est forte.

Pour atteindre la teneur admise par la spécification carburant (inférieure à 200 ppm), il est nécessaire de séparer la glycérine dissoute dans la phase ester.

Selon le type de procédé utilisé, cette séparation se fait différemment. Dans les procédés dits en catalyse homogène, tels que décrits par exemple dans le document EP-B1-0356317 ou WO 2007/034067, l'étape de purification peut se faire par lavage successifs à l'eau et passage sur des résines échangeuses d'ions.

Dans le procédé Esterfip-H™ développé par l'IFP mettant en oeuvre un catalyseur hétérogène, la séparation entre le glycérol et l'ester produit se fait en plusieurs étapes. En effet, le méthanol agissant comme co-solubilisant des esters méthyliques et du glycérol, l'étape d'évaporation de l'alcool réalisée en sortie de la section réactionnelle rend insoluble une partie du glycérol présent dans le flux à une teneur comprise entre 0,1 et 5% masse. La partie soluble représente à température ambiante 300 à 700 ppm masse, la teneur maximale admissible imposée par la norme européenne étant de 200 ppm masse de glycérol libre. Cela impose donc de séparer à la fois le glycérol insoluble et une partie du glycérol soluble. La séparation se fait dans un premier temps par décantation gravitaire dans un ballon décanteur. Le flux d'esters sortant du décanteur est ensuite envoyé dans un coalesceur. Le flux de phase de glycérine est soutiré en point bas du coalesceur. Des procédés permettant d'améliorer ces deux étapes de séparation ont été décrits dans les demandes de brevet déposées au nom de la Demanderesse sous le n° FR 07/04 711 et FR 07/04 715.

Toutefois, il est nécessaire de faire une purification finale par adsorption sur des solides, par exemple sur des résines échangeuses d'ions. À l'issue de cette étape, la teneur en glycérine de la phase ester répond ainsi à la spécification carburant (inférieure à 200 ppm).

Les résines échangeuses d'ions utilisées dans ces étapes de purification finale de la phase ester fonctionnent en alternant des cycles d'adsorption et de régénération.

Les solides adsorbants se trouvent donc en contact d'une partie de la glycérine insoluble. Les propriétés de ces solides sont telles qu'ils ont une affinité très forte avec la glycérine contenue dans le flux à purifier. Lorsque ce flux contenant majoritairement de l'ester, des glycérides partiellement convertis, des traces de méthanol, et de la glycérine soluble et insoluble va passer au travers du lit fixe de résines, le solide va capter c'est-à-dire retenir par un phénomène de "physisorption" la glycérine soluble et insoluble et laisser passer les autres molécules. Ce solide possède des sites qui par leurs configuration et/ou polarité vont attirer la glycérine et la retenir. Chaque site ne peut cependant capter qu'un nombre fini de molécule de glycérine, les autres présentes dans le flux d'ester passeront sans être retenues. Ainsi une molécule de glycérine présente dans le flux à purifier traversant le lit sera captée par le premier site non saturé qu'elle rencontrera, mais passera à travers le lit si elle n'en rencontre pas. De façon globale, tant que le lit ne sera pas saturé, il n'y aura pas de glycérine dans le flux obtenu en sortie des résines mais dès que le lit sera saturé, la teneur en glycérine dans ce flux augmentera jusqu'à devenir identique à celle du flux à purifier entrant (phénomène de percée). Il est alors nécessaire de régénérer les résines c'est-à-dire de retirer la glycérine captée par un solvant approprié.

De façon à assurer en permanence un flux produit sans glycérine, répondant aux spécifications biocarburants imposées par la norme européenne actuelle, la zone de purification est constituée de deux adsorbeurs fonctionnant de façon intermittente. Pendant que l'un est en phase d'adsorption, l'autre est régénéré de façon à disposer toujours d'un adsorbeur régénéré lorsque celui qui travaille en mode adsorption se sature.

Pour pouvoir assurer le respect continuel de la spécification en glycérine dans le flux d'ester final, il est donc essentiel de suivre très finement le moment du perçage, c'est-à-dire le moment où le lit est saturé et donc où la teneur en glycérine dans le flux produit augmente très rapidement et risque de dépasser la teneur en glycérine admise par la spécification carburant. Le temps de perçage c'est-à-dire le temps nécessaire pour passer d'une teneur quasi nulle en glycérine à la teneur du flux entrant est de l'ordre de 30 minutes dans le cas du procédé Esterfip-H™.

Il existe des méthodes d'analyses couramment utilisées pour déterminer la teneur en glycérine dans le flux sortant et donc pour détecter le moment du perçage. On peut notamment utiliser la chromatographie en phase gaz. Cette méthode décrite dans la norme EN 14214 demande une préparation de l'échantillon d'au minimum 15 à 30 minutes avant injection dans un chromatogramme puis l'analyse chromatographique proprement dite est réalisée et dure en moyenne 2 heures.

Une autre méthode consiste à réaliser le dosage de la glycérine avec du métapériodate. Cette réaction est basée sur une réaction chimique avec le métapériodate sous pH contrôlé, un des produits de la réaction étant ensuite dosé. Cette réaction est réalisée en phase aqueuse. Il faut donc réaliser sur l'échantillon d'ester une opération de lavage à l'eau pour extraire la totalité de la glycérine présente et après décantation réaliser le dosage de la glycérine dans la phase aqueuse. Même si le dosage peut être automatisé, l'enchaînement de ces opérations (lavage + décantation + dosage) pénalise la précision et font que cette méthode reste longue (entre 1 h30 et 2 heures).

Les méthodes et temps d'analyses actuellement utilisés ne sont pas compatibles avec un suivi fin c'est-à-dire une détection en temps réel du perçage des résines et plus généralement des lits d'adsorbants. En effet, cela suppose de multiplier des analyses coûteuses et dans le meilleur des cas de pouvoir changer d'adsorbeur trop tardivement après le perçage (1 h30 à 2 h après), ce qui implique une production d'un volume non négligeable de produit hors spécification.

Nous recherchons une méthode de détection et/ou de suivi très rapide, en temps réel, de la présence de glycérine dans la phase ester.

La présente invention propose une méthode de détection et/ou de suivi du perçage des lits d'absorbants ne présentant pas les inconvénients présentés ci-dessus et s'applique à des procédés de fabrication de biodiesel dans lesquels il est nécessaire de séparer la glycérine du produit obtenu.

### RÉSUMÉ DE L'INVENTION

La présente invention décrit une méthode simple et rapide de détection et/ou de suivi du perçage d'un lit d'adsorbant utilisé dans un procédé de purification d'un mélange d'esters et de glycérine.

L'invention décrit l'installation dans laquelle la méthode de suivi du perçage du lit d'adsorbant utilisé lors de l'étape de purification d'un flux d'esters alkyliques d'acides gras est mise en oeuvre.

### DESCRIPTION DES DESSINS

La Figure 1 donne une représentation schématique d'une partie du procédé Esterfip-H™ tel que décrit dans l'art antérieur.
La Figure 2 donne une représentation schématique de la partie du procédé Esterfip-H™ dans laquelle s'effectue le procédé de purification selon la présente invention.

### DESCRIPTION DÉTAILLÉE DE L'INVENTION

La présente invention décrit un procédé de purification d'un mélange d'esters et de glycérine sur au moins un lit d'adsorbant captant la glycérine, dans lequel le perçage du lit est détecté et/ou suivi par mesure de la quantité de glycérine présente dans le flux purifié par turbidimétrie, la purification du flux d'esters se faisant sur au moins deux lits d'adsorbants placés dans des réacteurs différents, l'un au moins étant en mode d'adsorption et l'un au moins étant en mode régénération.

Préférentiellement, le mélange d'esters à purifier est un mélange d'esters alkyliques d'acides gras provenant d'un procédé de transestérification d'une huile végétale ou animale et d'un monoalcool aliphatique.

Le procédé selon la présente invention permet de détecter pratiquement en temps réel le moment précis du perçage du lit d'adsorbant et par conséquent d'éviter la production d'un volume trop important de produit ne répondant pas aux spécifications carburant.

Cette méthode est simple à mettre en oeuvre et ne nécessite aucune préparation préalable. Il n'est en effet pas nécessaire, contrairement aux méthodes d'analyse utilisées jusqu'à présent, de prélever des échantillons. La mesure par turbidimétrie se fait directement sur une dérivation de l'installation dans laquelle le procédé est mis en oeuvre : elle permet d'avoir ainsi un résultat de mesure instantané.

La mesure de turbidimétrie est fondée sur l'atténuation d'un faisceau de lumière dont les longueurs d'onde sont essentiellement dans le visible mais peuvent aller au delà, notamment jusqu'au proche infra-rouge. Un faisceau lumineux, provenant d'une lampe et focalisé par un système de lentilles, traverse le milieu à analyser et est reçu par un système de détection se trouvant à l'opposé. Ce faisceau est traité par un filtre qui va sélectionner le domaine de mesure choisi (UV, visible ou IR). Une photodiode quantifie au final l'intensité lumineuse résiduelle. L'atténuation du faisceau de lumière incidente obéit à la loi de Beer Lambert, et la mesure de cette atténuation permet de connaître en continu la concentration d'un liquide, un changement de phase ou de mesurer une turbidité.

La mesure de turbidité utilise la propriété d'une particule non dissoute à dévier la lumière. Comme précédemment, un faisceau lumineux, provenant du module de transmission, va traverser le fluide se trouvant dans le volume de mesure et rencontrer d'éventuelles particules. Ces dernières vont dévier tout ou partie de ce faisceau. Des photodiodes, inclinées à un certain angle fixé par le constructeur de l'appareillage, vont mesurer l'intensité de lumière déviée et ainsi évaluer la concentration en particules. Une photodiode située à un angle de 0° pourrait être utilisée pour compenser en temps réel tout changement de couleur du liquide.

Le turbidimètre est étalonné préalablement à la réalisation de mesures, par toute méthode connue de l'homme de l'art et par exemple en effectuant des mesures de concentration par dosage au métapériodate.

La mesure de la quantité de glycérine présente dans le flux purifié est effectuée par turbidimétrie avantageusement après avoir refroidi le flux d'esters à une température d'au plus 20°C, de préférence d'au plus 10°C.

De manière surprenante, le procédé selon la présente invention permet de détecter une quantité de glycérine inférieure au seuil de solubilité classiquement admis puisque le turbidimètre détecte une quantité de glycérine inférieure à 200 ppm.

Les esters n'étant pas particulièrement polaires, la limite de solubilité du glycérol dans la phase ester est relativement basse comme le confirment certaines études de la littérature (D.S. Negi, F. Sobotka, T. Kimmel, G. Wozny, R. Schomäcker, Ind. Eng. Chem. Res., 2006, 45, 3693-3696, H. Zhou, H. Lu, B. Liang, J. Chem. Eng. data, 2006, 51, 1130-1135 et A.E. Andreatta, L.M. Casas, P. Hegel, S.B. Bottini, E.A. Brignole, Ind. Eng. Chem. Res., 2008, in press) menées sur des systèmes ternaires ester/méthanol/glycérol.

Cependant, la vraie limite de solubilité du glycérol dans les esters n'est pas facilement déterminable à partir des données publiées dans la mesure où les résultats reportés concernent des mélanges avec des quantités importantes de méthanol, jouant alors un rôle de co-solubilisant du glycérol dans l'ester et où le manque de points expérimentaux aux faibles teneurs en méthanol ne permet pas d'extrapoler les diagrammes ternaires à une valeur nulle de méthanol.

Il a donc été nécessaire de la déterminer. Pour cela, des mesures expérimentales ont été effectuées sur le système ester méthylique d'huile végétale de colza / glycérol. Les mesures de la solubilité réalisées consistent à mettre en présence de l'ester et du glycérol en excès de telle sorte qu'on obtienne deux phases distinctes et donc qu'on soit assuré d'avoir saturé la phase ester en glycérol, dans une étuve thermostatée à la température choisie pendant un temps suffisamment long pour que l'équilibre soit atteint. Puis, un prélèvement est effectué dans la phase ester et est analysé par chromatographie en phase gazeuse selon la méthode de dosage de glycérol dans l'ester décrite dans la norme EN14214. L'ensemble de ces valeurs est reporté dans le tableau 1 suivant.

**Tableau 1**

| **T (°C)** | **Cₗᵢₘᵢₜₑ(glycérol) (ppm)** |
|---|---|
| 20 | 320 |
| 43 | 484 |
| 61 | 813 |
| 80 | 1233 |

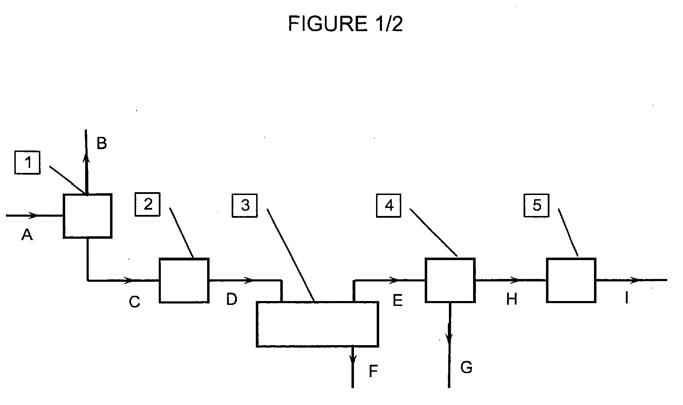

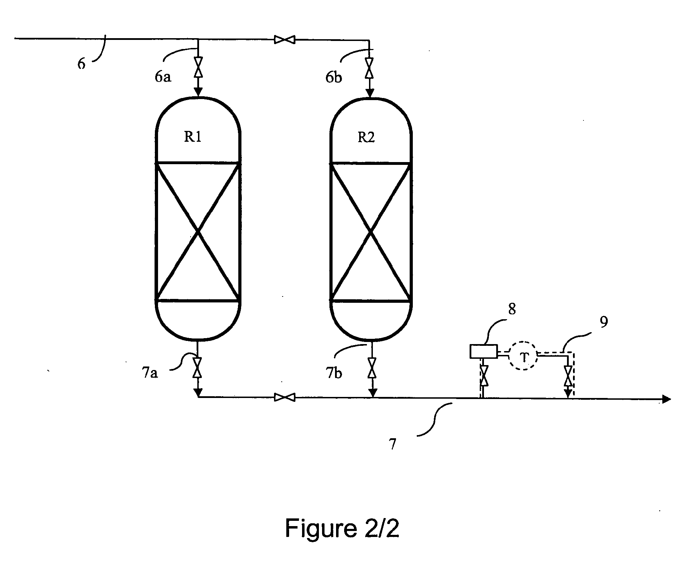

La présente invention peut être maintenant décrite en se référant aux Figures 1 et 2, relatives à un procédé de transestérification d'huiles végétales ou animales avec un monoalcool aliphatique.

Dans le procédé représenté schématiquement sur la Figure 1 et tel que décrit dans l'art antérieur, le flux A, en sortie de section réactionnelle, contient majoritairement des esters méthyliques, du méthanol, du glycérol et des glycérides partiellement convertis (monoglycérides, diglycérides et triglycérides), ainsi que des traces d'eau, impureté présente dans la charge. La zone (1) correspond à la zone dans laquelle s'effectue l'étape d'évaporation du méthanol, et la zone (2) est avantageusement un échangeur de chaleur. La séparation de la glycérine se fait dans une zone de séparation qui avantageusement opère en plusieurs étapes se faisant dans les équipements (3) à (5) correspondant respectivement à un ballon décanteur (3), un coalesceur (4) et une zone d'adsorption sur solides (5) (ou encore adsorbants), correspondant à la zone appelée "zone de purification" dans la présente invention.

La Figure 2 représente la zone (5) de purification dans laquelle la méthode de détection et/ou de suivi du perçage du lit d'adsorbant selon l'invention est mise en oeuvre et correspond à une amélioration du procédé Esterfip-H™ .

La purification du flux d'esters se fait sur au moins deux lits d'adsorbants placés dans des réacteurs différents, l'un au moins étant en mode d'adsorption et l'un au moins en mode régénération. Lorsque la mesure de la quantité de glycérine présente dans le flux en sortie d'un réacteur R1 fonctionnant en mode d'adsorption atteint la valeur de consigne, on fait passer ledit réacteur en mode régénération, et le flux d'esters entrant et à purifier est envoyé vers un autre lit d'adsorbant capable de fonctionner en mode d'adsorption.

Le flux d'esters alkyliques devant être purifié circule dans les conduites (6) puis (6a) si il doit être envoyé vers le réacteur R1 (respectivement (6b) si il doit être envoyé vers le réacteur R2).

Les résines couramment utilisées comme adsorbants sont des résines acides (groupements sulfoniques) sous forme sodique, macroporeuses, à base du copolymère styrène-divinylbenzène réticulé. L'interaction privilégiée entre les résines et les molécules d'adsorbat est l'établissement de liaisons hydrogène soit entre les atomes d'oxygène ou de soufre du groupement sulfonique de la résine et des atomes d'hydrogène des molécules d'adsorbat soit entre les cations sodium des résines et des hétéroéléments tels que les atomes d'oxygène des molécules d'adsorbat. Par conséquent, ce type de résine fixe surtout des composés polaires et plus particulièrement ceux possédant des fonctions OH. Elle est donc tout à fait adaptée à l'adsorption de glycérol d'autant qu'il est dans l'ester qui ne contient que des traces de compétiteurs tels que l'eau ou des alcools.

Les adsorbants utilisés dans les réacteurs R1 et R2 sont choisis parmi les résines échangeuses d'ions, de préférence acides et très préférentiellement sous forme sodique, le gel de silice, les alumines, les silice-alumines ou les charbons actifs possédant des groupements oxygénés à leur surface.

Tout type de solides mésoporeux, et de préférence macroporeux, fonctionnalisés de telle sorte qu'une affinité particulière s'établisse entre ses fonctions et les groupes OH de la glycérine de manière à la retenir préférentiellement et possédant une résistance mécanique suffisante, est envisageable.

A la sortie du ou des réacteurs R1 et R2, le flux d'esters, normalement purifié, est envoyé vers le pool carburant, par l'intermédiaire d'une conduite (7). Dans cette conduite, le flux d'esters est à une température d'environ 60°C.

Une conduite (9) est montée en dérivation à partir de la conduite (7). A l'intérieur de la conduite (7), des moyens de refroidissement peuvent être adaptés, si besoin, pour refroidir le flux d'esters purifiés par exemple. Ainsi, le flux d'ester peut être refroidi jusqu'à environ 20°C, de préférence jusqu'à environ 10°C.

Le turbidimètre T est placé en sortie du ou des réacteurs. Si le turbidimètre est placé à l'intérieur de la conduite (7), et la mesure peut être par conséquent effectuée sur un flux d'esters refroidi. La solubilité de la glycérine dans les esters alkyliques est inversement proportionnelle à la température (voir tableau 1). En diminuant la température dans la conduite (7), on accélère ainsi la possibilité de détecter la glycérine insoluble, et en abaissant le seuil de solubilité, le glycérine soluble est rendue détectable.

Les mesures réalisées à l'aide du turbidimètre à intervalle de temps réguliers permettent par conséquent de détecter le perçage des adsorbants et le chargement progressif du flux d'esters produits en glycérine. En effet, la présence de glycérine dans la phase ester modifie les caractéristiques optiques de l'ester. Localement, avant même l'apparition d'une gouttelette de glycérine, il se produit une sursaturation détectable avec le turbidimètre.

Ainsi, il existe une valeur seuil pour la concentration en glycérine dans le flux d'esters alkyliques d'acides gras à partir de laquelle le turbidimètre répond. Le turbidimètre ne donne aucun signal en deçà de cette valeur seuil. Une réponse est observée sur le turbidimètre à partir d'environ 60 ppm de glycérine présent dans le flux d'esters. Cette valeur seuil reste inférieure à la concentration en glycérine autorisée selon la norme actuellement en vigueur.

L'obtention d'une réponse sur le turbidimètre (valeur seuil atteinte) signifie que le lit d'adsorbant utilisé est saturé et est en fin de cycle : il est nécessaire de le passer en mode régénération, ce qui consiste à retirer la glycérine captée par un solvant approprié. Par conséquent, le flux d'esters alkyliques entrant et devant subir l'étape de purification est envoyé vers un autre lit d'adsorbant fonctionnant lui en mode d'adsorption.

Si la mesure effectuée par exemple en sortie du réacteur R1 fonctionnant en mode d'adsorption atteint ou dépasse la spécification, ou plus généralement la valeur de consigne donnée par l'exploitant et notamment dépasse la valeur de 200 ppm de glycérine imposée par la norme, on opère à un changement de lit : le réacteur R1 passe en mode régénération et le flux d'esters alkyliques entrant devant subir l'étape de purification est envoyé vers l'autre lit d'adsorbant R2 fonctionnant en mode d'adsorption.

Il est possible de convertir la mesure du turbidimètre en signal analogique pouvant déclencher automatiquement le changement de lit d'adsorbant, à partir d'un niveau seuil défini par l'utilisateur.

Ainsi, la méthode de suivi du perçage selon la présente invention est une technique très flexible, permettant de suivre, en continu si nécessaire, la concentration en glycérine dans le flux d'esters. L'opérateur peut faire autant d'analyses qu'il le souhaite, la fréquence d'acquisition pouvant être modulée à volonté. Ainsi, cette fréquence sera relativement faible en début de cycles de fonctionnement du lit d'adsorbant étant donné que le risque de perçage est faible. La fréquence sera en revanche quasi-continue lorsqu'on arrive en fin de cycle pour détecter finement le perçage.

Un autre avantage de la présente invention est donc l'amélioration de la qualité du produit en évitant les périodes hors spécification inhérentes au temps d'analyse. De plus, l'opération est ainsi fiabilisée en évitant d'éventuelles erreurs humaines liées à la prise d'échantillon, aux manipulations et dosages de produits lors des analyses. Elle permet également de gagner sur les coûts laboratoire et de supervision en allégeant la charge de suivi et d'analyse et sur l'exploitation de l'outil industriel car affiner le suivi du perçage permet d'optimiser le seuil de changement donc d'exploiter au mieux la capacité d'adsorption du solide tout en respectant la qualité souhaitée.

La présente invention décrit également une installation comprenant une zone de transestérification d'huile végétale ou animale par un alcool aliphatique, une zone d'évaporation de l'alcool présent dans l'effluent issu de la zone de transestérification, éventuellement une zone d'échange thermique, une zone de séparation de la glycérine incluant une zone de purification sur adsorbants, installation dans laquelle :
- une conduite (6) amène un flux H d'esters alkyliques d'acides gras et de glycérine ,
- la zone de purification comprend au moins deux lits fixes d'adsorbants placés dans au moins deux réacteurs R1 et R2, l'un au moins fonctionnant en mode d'adsorption et l'un au moins en mode de régénération,
- une conduite (7a,7b) en sortie des réacteurs sur laquelle on fait une dérivation par une conduite (9) d'une partie du flux d'esters et où on place un turbidimètre pour effectuer la mesure.
L'installation comprend en outre un dispositif (8) de contrôle de la température du flux de la conduite (9) situé en amont du turbidimètre (T) dans le sens de la circulation dudit flux.

Avantageusement, la conduite (9) est entourée d'un dispositif adapté au refroidissement du flux d'esters circulant à l'intérieur de ladite conduite.

L'installation peut comporter en outre un dispositif de déclenchement du changement de lit, ledit dispositif étant actionné lorsque le turbidimètre donne une mesure de la quantité de glycérine au moins égale à la valeur consigne.

### EXEMPLE

Un suivi de la concentration en glycérine dans l'ester produit selon le procédé Esterfip-H après passage sur des résines acides avec groupements sulfoniques de type Lewatit Monoplus SP 112 commercialisée par Lanxess (Bayer) se présentant sous forme de billes monodisperses a été réalisé en utilisant un turbidimètre de type TF16 fabriqué par le constructeur Optek. L'angle d'inclinaison des photodiodes est dans ce cas de 11°. La mesure ayant été faite par la prise d'échantillons à différents instants à partir d'un temps initial nous permet de suivre la qualité de cet ester. L'ensemble de ces mesures a été réalisé à température ambiante.

Afin d'étalonner les mesures obtenues, des mesures de concentration sont réalisées par la méthode connue de dosage au métapériodate.

Le tableau suivant résume les valeurs obtenues :

| Temps (h) | Concentration au Métapériodate (ppm) | Concentration au Turbidimètre (u.a.) |
|---|---|---|
| | | |
| T0 | 55 | 0 |
| T0 + 1 | 61 | 0.001 |
| T0 + 6 | 130 | 0.038 |
| T0 + 9 | 191 | 0.066 |

Nous observons qu'il y a un effet de seuil : le turbidimètre est aveugle jusqu'à une concentration voisine de la soixantaine de ppm. Ensuite, il mesure des valeurs bien significatives. Du fait que la spécification imposée par la norme européenne actuelle est de l'ordre de 200 ppm, l'utilisation d'un turbidimètre est donc tout à fait à même de suivre en continu la concentration de la glycérine dans l'ester et de pouvoir détecter un fin de cycle des résines pour basculer d'un lit d'adsorbant à l'autre.

## Revendications

1. Procédé de purification d'un mélange d'esters et de glycérine sur au moins un lit d'adsorbant captant la glycérine, dans lequel le perçage du lit est détecté et/ou suivi par mesure de la quantité de glycérine présente dans le flux purifié par turbidimétrie, la purification du flux d'esters se faisant sur au moins deux lits d'adsorbants placés dans des réacteurs différents, l'un au moins étant en mode d'adsorption et l'un au moins étant en mode régénération.

2. Procédé selon la revendication 1 dans lequel on effectue la mesure de la quantité de glycérine présente dans le flux purifié par turbidimétrie après avoir refroidi le flux d'esters à une température d'au plus 20°C, de préférence d'au plus 10°C.

3. Procédé selon l'une des revendications précédentes dans lequel l'adsorbant est choisi parmi les résines échangeuses d'ions, de préférence acides et très préférentiellement sous forme sodique, le gel de silice, les alumines, les silice-alumines ou les charbons actifs possédant des groupements oxygénés à leur surface.

4. Procédé selon l'une des revendications précédentes dans lequel, lorsque la mesure de la quantité de glycérine présente dans le flux en sortie d'un réacteur R1 fonctionnant en mode d'adsorption atteint la valeur de consigne, on fait passer ledit réacteur en mode régénération, et le flux d'esters entrant et à purifier est envoyé vers un autre lit d'adsorbant capable de fonctionner en mode d'adsorption.

5. Procédé selon la revendication 4 dans lequel la mesure du turbidimètre est convertie en signal analogique et déclenche automatiquement le changement de lit.

6. Procédé selon l'une des revendications précédentes dans lequel le mélange d'esters à purifier est un mélange d'esters alkyliques d'acides gras provenant d'un procédé de transestérification d'une huile végétale ou animale et d'un monoalcool aliphatique.

7. Installation comprenant une zone de transestérification d'huile végétale ou animale par un alcool aliphatique, une zone d'évaporation de l'alcool présent dans l'effluent issu de la zone de transestérification, éventuellement une zone d'échange thermique, une zone de séparation de la glycérine incluant une zone de purification sur adsorbants, installation dans laquelle :
- une conduite (6) amène un flux H d'esters alkyliques d'acides gras et de glycérine ,
- la zone de purification comprend au moins deux lits fixes d'adsorbants placés dans au moins deux réacteurs R1 et R2, l'un au moins fonctionnant en mode d'adsorption et l'un au moins en mode de régénération,
- une conduite (7a,7b) en sortie des réacteurs sur laquelle on fait une dérivation par une conduite (9) d'une partie du flux d'esters et où on place un turbidimètre pour effectuer la mesure.

8. Installation selon la revendication 7 comprenant en outre un dispositif (8) de contrôle de la température du flux de la conduite (9) situé en amont du turbidimètre (9) dans le sens de la circulation dudit flux.

9. Installation selon la revendication 8 dans laquelle la conduite (9) est entourée d'un dispositif adapté au refroidissement du flux d'esters circulant à l'intérieur de ladite conduite.

10. Installation selon l'une des revendications 7 à 9 comportant en outre un dispositif de déclenchement du changement de lit, ledit dispositif étant actionné lorsque le turbidimètre donne une mesure de la quantité de glycérine au moins égale à la valeur consigne.
